# EUROPEAN PATENT SPECIFICATION

(11) **EP 1 246 934 B1**
(45) Date of publication and mention of the grant of the patent: **17.11.2004**
(21) Application number: 00985593.3
(22) Date of filing: 15.12.2000
(51) Int. Cl.: C12Q 1/18

(54) **MICROBIAL GROWTH VARIATIONS**
MIKROBENWACHSTUM-VARIATIONEN
VARIATIONS DE CROISSANCE MICROBIENNE

(30) Priority: 16.12.1999 GB 9929808
(43) Date of publication of application: 09.10.2002
(73) Proprietor: Carr, Anthony Hugh, Stevington, Bedforshire MK43 7QB (GB)
(72) Inventor: Carr, Anthony Hugh, Stevington, Bedforshire MK43 7QB (GB)
(74) Representative: Thornton, Neil
(86) International application number: PCT/GB2000/004842
(87) International publication number: WO 2001/044498

(56) References cited:
- WO-A-97/37036
- GB-A- 1 554 134
- US-A- 4 236 211
- US-A- 5 759 799

## Description

### FIELD OF THE INVENTION

This invention centres on microbiological test processes, in particular those involving testing of the influence of antimicrobial compounds, nutrients and vitamins or other growth promoters. In particular the invention is concerned with lack of uniformity in response to such agents.

In the clinical laboratory it is often necessary to isolate pathogenic organisms from body fluid samples derived from patients. When found such organisms are frequently further tested to establish their sensitivity to antimicrobial substances, and thus influence the choice of therapy for the specific patient.

Numerous methods have been developed, with a requirement where possible to reduce the time taken to derive a result. To date many so called rapid methods have frequently been disrupted by an inability to detect latent antibiotic resistance. This raises clinical reservations as to the efficacy of such methods in determining treatment of patients. In general terms the majority of organisms will be suppressed by some minimum concentration of antibiotic, and are thus inhibited. Antibiotic resistance, when present, can be associated with a sub population which, for one reason or another, are not inhibited.

Accordingly it is the purpose of the present invention to provide a new test process whereby differences in growth responses are detected. In particular the presence of any antibiotic resistant sub populations can be established within the duration of the test.

### BACKGROUND TO THE INVENTION

Infectious diseases remain one of the major causes of morbidity and mortality in the human race. Microbiological test processes, especially microbial identification and antibiotic sensitivity, are of paramount importance to both the individual and society as a whole.

Clinical laboratories often test microbes clinically isolated from patient and other sources, examining their sensitivity to antimicrobial agents and their metabolic requirements. Effective antibacterial therapy is fundamental to modern medicine, but equally the ability to treat infectious diseases is often taken for granted.

An early method used the standard petri-dish/agar growth medium with the addition of paper discs impregnated with various drugs. Antibiotic diffuses out of the paper carrier to create a circular zone. If the organism is sensitive then a clear area, or zone of inhibition, will be observed after overnight incubation. Later tests became more quantitative, using a series of tubes containing doubling concentrations of an antibiotic. Again after incubation growth was evident, in this case as turbidity, in those tubes which failed to inhibit. This technique provides an indication of the Minimum Inhibitory Concentration [M.I.C.]. Subsequently the tube method was miniaturised by adaptation of the 96-well serology plate to provide one 8 x 12 matrix of tests. In this case overnight incubation causes growth to appear as a 'button' or deposit at the base of the well.

A large number of methods and types of apparatus have been developed to carry out such tests, not just involving antibiotics but also nutrients, growth factors and other inhibitory agents. Examples of plates and their use are found in GB 1,574,707 (Unilever) French Patent 1,488,866 (Sclavo) and others.

In the whole area of antibiotic sensitivity testing it is desirable to minimise the time before the result can influence treatment. It is also desirable to automate the test processes as far as possible. Rapid methods are defined as of short duration, in the clinical context this is usually interpreted as within the same working day. In order to achieve more rapid tests microbiological processes could no longer rely on 18-24 hour [overnight] incubation to a visual end point. The measurement of growth became essential as seen in US patent 4,236,211 and GB patent 1,554,134 where light scattering by micro-organisms served as a detection method. Widespread adoption of microdilution plate methods provided convenient, stable ready-made test panels. The quest for rapid results caused the incorporation of chromogenic or fluorogenic substrates, linked to optical reading systems. Increased microbial activity associated with growth, caused the appearance of colour or fluorescence. An early, very effective version of this approach is described by Staneck J.L et al [J. Clin. Micro. (85)22(2)187-191]. The commercially available "Sensititre"™ product combined fluorogenic substrates with the microdilution format in both sensitivity testing and microbial identification.

The emergence of rapid systems, and increasing experience in comparative studies with traditional methods, became associated with anomalies which sometimes occur. Rapid [early] results could give low MIC values which were not supported by overnight [late] results. These tend to be most obvious in systems which offer continuous monitoring or dual reading at both early and late intervals. Empirical explanations linked such anomalies to inoculum concentration, enzymatic destruction of antibiotic and antibiotic quantity per cell. A common recommendation was increased inoculum levels from 10⁵ to 10⁶ cfu/ml., and this was also technically attractive because the sensitivity of growth detection is less demanding. A frequently reported anomaly was due to β lactamase activity in Staphylococcus aureus, so much so that EP 84301273 describes a rapid test for β lactamase activity. When added to the "Sensititre" panel this test provided a warning that a low early MIC could be misleading. When more extensive data accumulated it became apparent that the problems of rapid tests were far more substantial than initial indications suggested. It also became apparent that each of the commercially available systems had very individual characteristics [in which the main influences were working volume, inoculum level and type of technology used to assess growth].

The microbiological explanation for these anomalous results is the presence of very small, sub-populations of resistant cells. Essentially the abilities of rapid tests are limited to the response of the main population. In contrast the protracted, overnight incubation can allow this small population to proliferate and register as detectable growth. If therapy were to be based on the early result then antibiotic would be prescribed at modest levels. The implications are that not only would the treatment be ineffective against the sub-population, but could select for such cells and thus exacerbate the situation. The proliferation of modern antibiotics has been mirrored by a very diverse range of resistance mechanisms. Antibiotics do exercise a selection pressure and it is almost inevitable that resistant bacteria will evolve and thrive. The physician, finding patients failing to respond to antibiotics, will rightly lack confidence in the laboratory method declaring the organism fully sensitive.

Eventually the difficulties were attributed to the incidence of sub-populations, at relatively low levels, where antibiotic resistance destroyed the efficacy of rapid antibiotic sensitivity/MIC determinations. There is a fine balance between effective antimicrobial therapy and failure to treat an infection because of microbial resistance mechanisms. The examples of organism resistance combinations are legion, with the following being by no means comprehensive:
Gram -ve resistance to Amikacin, Netilmycin, tobramycin
Gram -ve resistance to third generation Cephalosporins due to extended spectrum β lactamases [e.g. Klebsiella spp]
G +ve pneumococci resistance to penicillins
G +ve staphylococci resistance to methicillin
Group A streptococci resistance to erythromycin [Europe/Japan] Enterococci resistance to vancomycin and aminoglycosides.

Two previously marketed systems - Autobac™ and Abbott MS-2™ or Advantage are no longer available.

Sensititre™, although among the popular microdilution products, is limited to 18-hour operation. Two other systems are described in publications as dealing with these issues somewhat tangentially. The Bio-Merieux Vitek™ is reported as incorporating an expert system to analyse atypical patterns and report the most likely resistance mechanisms present in an isolate, using the Antibiogram™. The Baxter MicroScan™ is said to feature software to alert the pharmacy to patients on inappropriate or excessive therapeutic choices.

It is clear that all previous rapid methods have definite limitations, equally the inability to detect resistance has created a situation in which method changes are marginal rather than any major update to the technology. Inevitably attention has moved to Genotype determinations looking at resistance detection at the genetic level, with some scope for interpretative reading of antibiotic sensitivity tests. Applications of the two major techniques [DNA hybridisation and Polymerase Chain Reaction P.C.R.] are reviewed in Courvalin P. [ASM News(1992)58:369-75]. The barriers to the use of such methods are as follows:
I. Multitude of unrelated resistance mechanisms
II. Resistance not necessarily expressed
III. False positives from dead or commensal cells
IV. Complex and costly methods

There are some ideal applications, especially among slow growing organisms where a specific resistance mechanism is suspected and inappropriate therapy has major implications [e.g. Mycobacterium tuberculosis]. Genotypic methods can provide invaluable information when it is not easily available by other means.

In contrast Phenotypic methods should be inexpensive and simple, all mechanisms are detected and there is established data linking tests results to clinical outcome. These methods are far more flexible in the range of antibiotics used. Where growth rate determinations are a key element it is important to limit the impact of factors such as media composition, temperature and atmosphere composition.

The main technical barrier is the improvement in the sensitivity of growth detection technology and differentiating resistant sub-populations. In this respect existing test systems are severely limited. Interpretative methods depend very heavily upon a substantial database on antibiotics and resistance which in turn calls for complex logic to apply information effectively. The phenotypic approach of measuring activity of antibiotics against cultures of clinical isolates remains necessary.

Expression of resistance at low levels is the challenge because it is essential that phenotypic tests can distinguish between the sensitive population and resistant strains.

MIC Determination is neither more nor less than an attempt to predict the result of antibiotic treatment of an infected patient. When most of the simple variables are controlled, and human error is minimised, it becomes apparent that the assumption that all bacterial cells in a population are the same is a simplistic view - biological variation is, above all, of paramount importance. The examination of growth variations and differentials, in the presence of any set of conditions [such as determinate antibiotic levels] is essential. This invention removes limitations inherent to current antibiotic sensitivity tests, with the potential to supersede MIC methods by giving a more definitive test result and yielding more information in interpretation.

### DRAWINGS

### Figure 1 a-d

Results of the method performed using Staphylococcus aureus and penicillin from Example 1-4.

### OUTLINE OF THE INVENTION

It is the purpose of the present invention to apply high sensitivity monitoring to microbial growth patterns during the stages of incubation. It is another purpose of the present invention to monitor such growth patterns across numerous replicates of any given test situation [e,g, ostensibly identical test conditions consequently differential effects are directly attributable to the organisms present]. It is still another purpose of the present invention to monitor such growth patterns across replicates and within a series of tests [e.g. doubling concentrations of antibiotic as in an MIC determination]. It is still another purpose of the present invention to estimate the frequency, if any, of atypical growth responses within each of a series of tests.

It is yet another purpose of the present invention to characterise the differentiating features of each and any such variation in growth responses, in order to provide additional information on the likely cause of such variation.

Finally another purpose of the present invention is to report each growth response and emphasise any detectable heterogeneity arising for any reason. The ability to obtain useful early/rapid results, particularly in antibiotic sensitivity tests, has obvious clinical benefits. In addition the detection of any low grade resistance enhances the quality of the result and reinforces the efficacy of the test process as a laboratory diagnosis.

### INVENTION

The majority of existing tests provide a regime in which each test condition [e.g. antibiotic, vitamin or nutrient concentration] is present as a single test within a series. In a microdilution MIC determination each well is inoculated with approx. 5 x 10³ organisms [colony forming units c.f.u.]. Since these products demonstrate early/late anomalies there are apparently sufficient cells present to express the effect. Equally there are finite limits to the number available after colony selection from the primary plate. For these reasons it seems reasonable that any new test deals with approximately equivalent numbers, although there are advantages in increasing the numbers to enhance detection of atypical cells.

In a given set of conditions and nutrient supply micro-organisms can be expected to grow and thus increase in numbers. If, for example, an organism has a doubling time of 30 mins then after an initial delay [lag] each cell will divide to create two cells at this interval. Within the short term - say, 3 hours - a single cell will yield in excess of 50-100 cells. It follows that a larger number - say 100 cells - can in the same time span generate about 5 x 10³ cells. In contrast after a protracted incubation organisms will reach a maximum number which can be supported by the available nutrients, regardless of the number from which the final population originated. It is important to note that the differential is only of value during the transient stages of early incubation. During incubation the response of organisms to an antibiotic will depend on concentration and mode of action. Growth patterns will generally fall into these categories:
I. Positive control [no drug] maximum growth rate
II. Viable, no change in numbers - bacteriostasis
III. Cell death - bactericidal action
IV. Partial inhibition - impaired, reduced growth rate
V. No real increase but aberrant growth - e.g. cell elongation.

This invention moves away from conventional approaches by seeking to emphasise and detect any inherent variability. To do this each test condition [e.g. antibiotic concentration] is present as a large number of replicates. These are inoculated at low levels, with what amounts to the organism loading of the single test subdivided between replicates. This strategy exacerbates any biological variation since some wells may include atypical cells [e.g. resistant].

Within a given test condition [e.g. antibiotic concentration] the growth responses can be compared and contrasted across all replicates [also on a time course basis by regular monitoring where available]. Any growth [increase in numbers] must be looked at in the context of all replicates. A general, gross increase across most replicates suggests the whole population is actively growing. In contrast a random pattern of small increases, in a proportion of replicates, suggests that such growth is arising from a low level distribution of resistant sub-population. This will only be evident where the main population is inhibited. When applied to MIC Determinations the 'uniform' isolate will provide an uncomplicated, consistent result, repeated numerous times in the sets of replicates. In contrast the culture with low level resistance will exhibit significant differential growth effects, related to the incidence and distribution of resistant cells across the test array.

The ability to follow and compare growth responses depends on sensitive detection methods. It is also of value to establish baselines by averaging multiple results in any given test situation. Comparative methods are able to pinpoint atypical responses using the majority as a control. The reduction in inoculum level reduces any tendency for the sub-population to be masked by the density of the main inoculum. In the simplest form the assessment of growth is essentially growth/no growth. During the early stages of incubation any growth is evident as a progressive increase in numbers. Each species will tend to grow at some maximum rate associated with the species itself and the conditions of the test. Any antibiotic present will tend to decrease the growth rate, usually in direct proportion to the concentration of antibiotic.

A quantitative assessment of growth adds a further dimension to the test process. Comparison between a culture containing no antibiotic and those subjected to known levels provides additional information. The extent to which a culture is impaired is a valuable indicator as to the concentration of drug needed to achieve effective inhibition.

It is well established that test processes involving incubation of a culture become easier to monitor the longer growth takes place. The ability to increase in numbers provides amplification of results, conversely the greater the sensitivity of the microbial detection then the earlier results become available. When combined with high sensitivity detection the test process described within this invention provides a rapid diagnostic test of significant clinical value. In contrast with previous test systems the process described here takes into account low grade resistance, thus providing the clinician with accurate and comprehensive information.

Observations of growth may be made between 6-12 hours. Observations of growth may be made between 18-24 hours. Periodic observations [semi-continuous], or continuous monitoring, may be carried out for at least 18-24 hours to automatically assess growth patterns.

When considering the ability to estimate the incidence of resistant responses, and hence rank the proportion of sub-population, there is an analogous technique. The Most Probable Number MPN Index [McCrady MH(1915) J.Inf.Dis.(12) 183] is not an accurate enumeration but does provide an indication [precision influenced by number of replicates]. Where an automated reading system is incorporated this type of data would be gathered rapidly, in which case an algorithm could be applied to monitor the status of any resistant sub-population. There is also a possibility that repeat testing would detect any escalation or increase in severity.

So far this text describes the detection and estimation of incidence levels for a resistant sub-population - as an example of biological variation expressed within an antibiotic sensitivity test. This could be more distinctive where variation is true heterogeneity - i.e. more than one species is involved. Under such circumstances the basic principles need refinement to incorporate additional qualitative criteria. At this stage very limited examples are possible - for example using epifluorescence observation as a detection system.

It is possible to visually distinguish Gram -ve rods from Gram positive cocci, consequently growth responses can be assigned accordingly. In a model system the results simply resemble the two individual MIC's superimposed. A test of this type would be complicated by any antagonism or synergy in the mixed culture situation. Although very limited this does suggest that direct tests on mixed cultures could take place. Operation of a test in this way includes the basic assessment of growth, as described, but with additional characterisation. Qualitative information [physical properties, reaction with reagents, biochemical activity or some other cytochemical factor] could serve to distinguish between species in a polymicrobic culture. Any growth produced would be ranked pure for a particular species, or mixed as inoculated. This approach exploits the selectivity of antibiotics as a potential mechanism for separating organism responses. The avoidance of the 24 hour primary/isolation plate would represent a time saving but in reality the interpretation, clinical value and acceptance are far from established.

The basic principle of the current invention is the comparison of differential growth patterns, to detect and rank variations.

Exploitation of the method would benefit from repackaging into purpose designed test arrays. Modifications and improvements will occur to those skilled in the art. In particular non-invasive, semi-continuous growth monitoring would simplify operation of the test system.

The method can determine the presence or absence of drug resistant bacteria, for example, and therefore can also be used to monitor the changes in drug resistant bacteria over a period of time. By repeating the method and comparing the actual or relative numbers of the major and minor bacterial populations any changes in population groups can be tracked and the effectiveness of any treatment assessed.

We desire it to be understood, therefore, that this invention is not limited to the particular forms shown. These are convenient to demonstrate principles but we intend to encompass modifications that do not depart from the spirit and scope of this invention.

### EXAMPLES 1 - 4

*Format:* In general terms the entire series of tests followed the methods for use of microdilution plates. Each of the eight antibiotic concentrations was represented by 32 replicate wells, thus amounting to 8 x 32[256] test wells in a three plate array.
*Antibiotic:* Penicillin
*Organisms:* Two cultures of Staphylococcus aureus, with one isolated from plates containing penicillin supplemented agar to provide cells of known β lactamase activity.
*Inoculum:* A target level of 10² cells/wells was selected to test an adequate number of organisms within each treatment. In the examples 2 - 4 the aim was to include an increasing proportion of resistant/β lactamase positive cells. Preparation by dilutions at these levels will undoubtedly be prone to errors.
*Incubation:* Sealed plates incubated for 5 hours at 37°C [previously shown to provide significant growth]. At the end of incubation well contents [50µl] were transferred to 200µl using individual sterile pipette tips [i.e. one/well].

### Growth assessment

### i. Viability in culture

A 100µl sample of diluted well contents was plated and spread on culture plates. The growth, corresponding to the situation at 5 hours, was assessed after incubation of the culture plates overnight.

### ii. Direct Epifluorescence Method

A second 100µl sample was filtered through a commercially available 0.2µ nuclepore membrane. This non-fluorescent, black filter was supported on a 5.0µ cellulosic membrane filter. Discrete 3.0mm diameter filter zones were created by attaching pre-punched black adhesive tape to the membranes. After filtration the zones were stained with 0.01% w/v Acriflavine™ in phosphate buffer at pH 7.2 [filtered immediately before use]. Direct observation, using an epifluorescence microscope resembles the DEFT method described by Pettifer GL Rodrigues UK [1982] J.Appl.Bact.53:323-329.
Using a large numerical aperture x 45 objective it is relatively simple to rank the estimated density of growth.
The viability test depicts growth responses in terms of organisms recovered and subsequently able to produce colonies on a secondary culture plate. Direct epifluorescence/filtration does constitute rapid detection but in this manual form is both cumbersome and labour intensive. Within these constraints both methods served to assign rankings to the growth response of every well. The two methods were adequate to indicate the presence of growth.

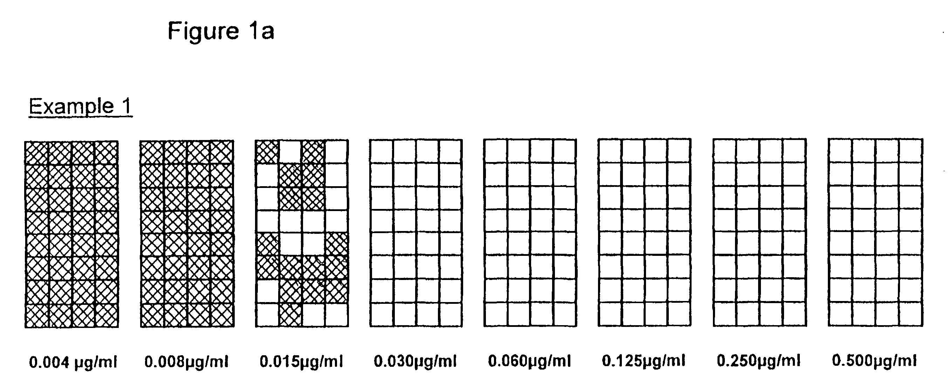

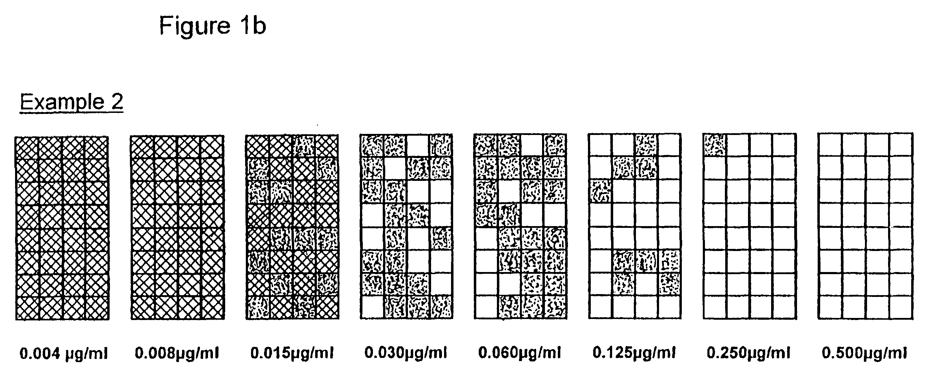

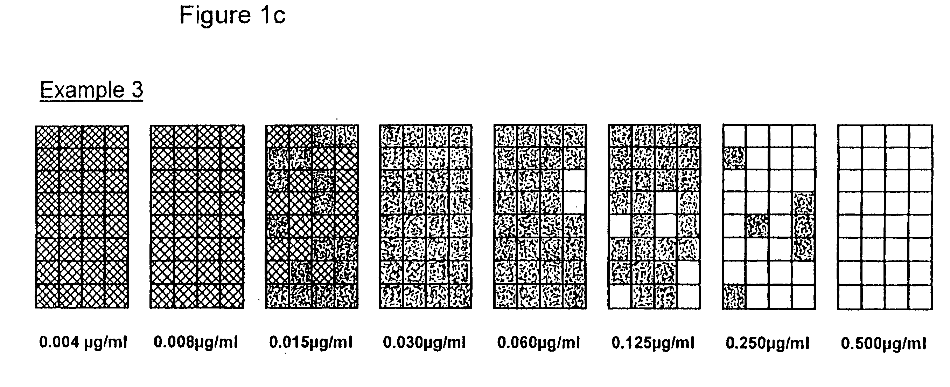

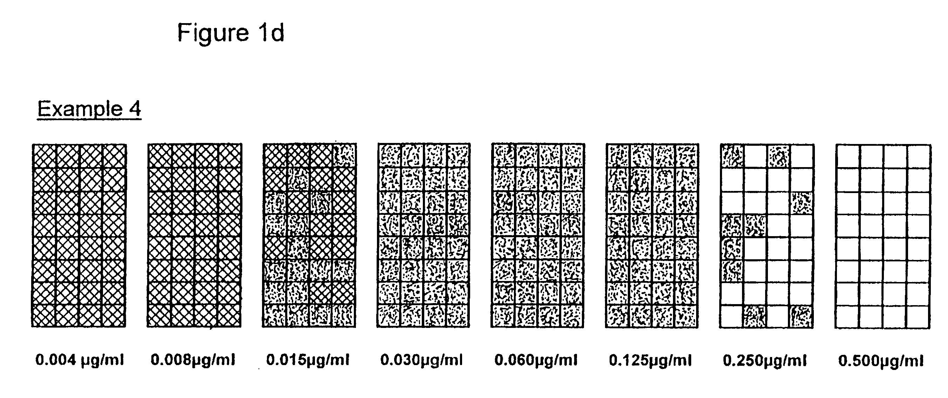

Figure 1 shows the results of Examples 1-4. It can be seen that at very low antibiotic concentration [e.g. 0.004µg/ml] all 32 replicate wells exhibit continued growth [dark shading]. With increasing concentration of antibiotic the population in some wells exhibits sensitivity [no shading], as seen at 0.015µg/ml. In Example 1 there is no evidence of growth in wells containing antibiotic at levels higher than 0.015µg/ml.

In Examples 2,3 and 4 an appreciable number of wells show low grade growth [light shading]. This is presumably attributable to the growth produced by a very small number [typically one] of resistant cells. Within a rapid timescale there will inevitably be a difference between the growth yielded by a whole inoculum multiplying compared to a few or even individual cells. In a longer period of incubation this differential is in fact lost. Examples 2,3 and 4 show increasing incidence of resistant cells; this corresponds to the proportions added during preparation of inocula. Dilution errors at these levels make it difficult to calculate actual ratios but the maximum tested here [Example4] probably corresponds to less than 10% of the population. The test examples demonstrate that, in Examples 2,3 and 4, there are resistant sub-populations in some wells which can be insensitive even at an antibiotic concentration of 0.250µg/ml.

Existing microdilution methods tend to use 50 microlitres added to each test well, where any specific antibiotic is present at one specific concentration in such a well [usually as part of a series].

With a microbial suspension at 10⁵ c.f.u./ml. This amounts to 5 x 10³ c.f.u./well.

In order to effectively detect small increases in numbers one would prefer a modest level of cellular background so that growth of any sub-population is distinguishable [i.e. is a significant proportion of test contents]. In a theoretical case the ideal would be 1 cell per sub-test [i.e. grow/not grow according to sensitivity to drug], but this would suggest a need for 5000 sub tests [probably impractical]. At the other end of the scale it might be possible to operate at 1000 cells in, say, 5 unit tests. A reasonable compromise range might be between 50 cells [in 100 replicate tests] to 500 cells [in 10 replicates], with a preferred target level of 100-200 cells in each of 50 replicate tests.

Preferably the number of colony forming units in each replicate test is in the range 25-500, preferably 100-200.

Preferably the number of replicates is between 20-100, typically about 50.

Depending on the test carrier/plate used in the method of the invention, the initial volume and/or initial concentration of organisms in the inoculum can be adjusted.

Also, the number of replicates can be increased above the number exemplified herein, in order to detect very low incidence rates for resistant cells.

## Claims

1. A method for determining the influence of a test substance on microbial growth, which includes the steps of:
a) taking a sample inoculum containing microbes whose growth response to the substance is to be tested, wherein the number and concentration of colony forming units of the microbe in the inoculum is approximately known;
b) dividing the inoculum into a plurality of replicates so that the total number of c.f.u.'s is distributed between the replicates;
c) contacting each of the replicates with the test substance under the same incubation conditions to provide replicate tests;
d) observing the influence of the substance on the growth of the microbes at one or more times during the incubation period;
e) differentiating the typical growth response of a majority of microbes constituting a main population group from a typical growth response of a minority of microbes constituting a minor sub-population group, the differentiation being on the basis of heterogeneity of response between respective replicate tests.

2. A method according to claim 1 wherein the test substance is an antibiotic.

3. A method according to claim 1 wherein the test substance is a bacterial nutrient.

4. A method according to any preceeding claim wherein the growth response is selected from the group consisting of:
I. growth rate change
II. bacteriostasis
III. bactericidal action
IV. total or partial growth inhibition
V. aberrant growth characteristics.

5. A method according to claim 2 wherein the inoculum is distributed among replicate tests, at each antibiotic concentration, in such a way as to obtain a defined number of colony forming units in each and every replicate.

6. A method according to claim 5 wherein the number in each test is in the range 25-500, preferably 100-200.

7. A method according to claim 5 or 6 wherein the number of replicates is between 20-100, typically about 50.

8. A method according to claim 1 wherein an observation of the growth is made at about 5 hours of incubation.

9. A method according to claim 1 wherein observations of growth are made between 6-12 hours.

10. A method according to claim 1 wherein observations of growth are made between 18-24 hours.

11. A method according to claim 1 wherein periodic semi-continuous observations, or continuous monitoring, are carried out for at least 18-24 hours to automatically assess growth patterns.

12. A method according to claim 11 wherein first results are presented as soon as significant differential effects are detected.

13. A method according to claim 2, wherein the differentiation in growth response of the microbial populations indicates a population of drug resistant microbes.

14. A method according to claim 3, wherein the differentiation in growth response of the microbial populations indicates a population of microbes in a favourable nutrient for growth.

15. A method according to any of the preceeding claims, wherein the differentiation between the main population group and the minor sub-population group is determined and recorded, the method is repeated and the differentiation between the population groups is compared with one or more previous results to determine whether or not there has been any change in either or both microbial population.

## Patentansprüche

1. Verfahren zur Bestimmung des Einflusses einer Testsubstanz auf mikrobielles Wachstum, umfassend die folgenden Schritte:
a) Entnehmen einer Impfmaterialprobe, die Mikroben enthält, deren Wachstumsreaktion auf die Substanz getestet werden soll, wobei die Anzahl und Konzentration koloniebildender Einheiten der Mikroben im Impfmaterial in etwa bekannt ist;
b) Aufteilen des Impfmaterials in eine Mehrzahl von Replikaten, so dass die Gesamtzahl von koloniebildenden Einheiten zwischen den Replikaten verteilt ist;
c) Inkontaktbringen der jeweiligen Replikate mit der Testsubstanz unter denselben Inkubationsbedingungen, um Replikattests bereitzustellen;
d) Beobachten des Einflusses der Substanz auf das Wachstum der Mikroben zu einem oder mehreren Zeitpunkten während der Inkubationsperiode;
e) Differenzieren der typischen Wachstumsreaktion einer Mehrheit von Mikroben, die eine Hauptpopulationsgruppe bilden, von einer typischen Wachstumsreaktion einer Minderheit von Mikroben, die eine untergeordnete Subpopulationsgruppe bilden, wobei die Differenzierung auf der Basis einer Heterogenität der Reaktion zwischen jeweiligen Replikattests erfolgt.

2. Verfahren nach Anspruch 1, wobei die Testsubstanz ein Antibiotikum ist.

3. Verfahren nach Anspruch 1, wobei die Testsubstanz ein Bakteriennährstoff ist.

4. Verfahren nach einem der vorherigen Ansprüche, wobei die Wachstumsreaktion ausgewählt wird aus der Gruppe bestehend aus:
I. Wachstumsratenänderung
II. Bakteriostase
III. Bakterizidwirkung
IV. totale oder teilweise Wachstumshemmung
V. abweichende Wachstumseigenschaften.

5. Verfahren nach Anspruch 2, wobei das Impfmaterial unter Replikattests, bei jeder Antibiotikumkonzentration, in einer solchen Weise verteilt wird, dass eine definierte Anzahl koloniebildender Einheiten in jedem einzelnen Replikat erhalten wird.

6. Verfahren nach Anspruch 5, wobei die Anzahl in jedem Test zwischen 25 und 500, vorzugsweise zwischen 100 und 200 liegt.

7. Verfahren nach Anspruch 5 oder 6, wobei die Anzahl der Replikate zwischen 20 und 100, typischerweise bei etwa 50 liegt.

8. Verfahren nach Anspruch 1, wobei eine Beobachtung des Wachstums nach etwa 5 Stunden Inkubation erfolgt.

9. Verfahren nach Anspruch 1, wobei Beobachtungen des Wachstums zwischen 6 und 12 Stunden erfolgen.

10. Verfahren nach Anspruch 1, wobei Beobachtungen des Wachstums zwischen 18 und 24 Stunden erfolgen.

11. Verfahren nach Anspruch 1, wobei periodische, halbkontinuierliche Beobachtungen oder eine kontinuierliche Überwachung für wenigstens 18 bis 24 Stunden durchgeführt wird/werden, um Wachstumsmuster automatisch zu bewerten.

12. Verfahren nach Anspruch 11, wobei erste Ergebnisse vorgelegt werden, sobald signifikante differentielle Effekte nachgewiesen werden.

13. Verfahren nach Anspruch 2, wobei die Differenzierung der Wachstumsreaktion der mikrobiellen Populationen auf eine Population arzneimittelresistenter Mikroben hinweist.

14. Verfahren nach Anspruch 3, wobei die Differenzierung der Wachstumsreaktion der mikrobiellen Populationen auf eine Population von Mikroben in einem günstigen Wachstumsnährstoff hinweist.

15. Verfahren nach einem der vorherigen Ansprüche, wobei die Differenzierung zwischen der Hauptpopulationsgruppe und der untergeordneten Subpopulationsgruppe bestimmt und aufgezeichnet wird, das Verfahren wiederholt und die Differenzierung zwischen den Populationsgruppen mit einem oder mehreren vorherigen Ergebnissen verglichen wird, um zu ermitteln, ob es bei einer der oder beiden mikrobiellen Populationen eine Veränderung gegeben hat oder nicht.

## Revendications

1. Procédé pour déterminer l'influence d'une substance de test sur la croissance microbienne, qui comprend les étapes consistant à :
a) prendre un agent d'inoculation échantillon contenant des microbes dont la réponse de croissance à la substance doit être testée, où le nombre et la concentration d'unités formant des colonies du microbe dans l'agent d'inoculation, sont approximativement connus;
b) diviser l'agent d'inoculation en une pluralité de réplicats de sorte que le nombre total de CFU est distribué entre les réplicats;
c) mettre chacun des réplicats en contact avec la substance de test dans les mêmes conditions d'incubation pour donner des tests en réplicat;
d) observer l'influence de la substance sur la croissance des microbes à un ou plusieurs moments durant la période d'incubation;
e) différencier la réponse de croissance type d'une majorité de microbes constituant un groupe de population principale, d'une réponse de croissance type d'une minorité de microbes constituant un groupe de sous-population mineure, la différenciation étant sur la base de l'hétérogénéité de réponse entre des tests en réplicat respectifs.

2. Procédé selon la revendication 1, dans lequel la substance de test est un antibiotique.

3. Procédé selon la revendication 1, dans lequel la substance de test est un nutriment bactérien.

4. Procédé selon l'une quelconque des revendications précédentes, dans lequel la réponse de croissance est sélectionnée parmi le groupe consistant en :
I. changement dans le taux de croissance
II. bactériostase
III. action bactéricide
IV. inhibition de croissance totale ou partielle
V. caractéristiques de croissance abérrante

5. Procédé selon la revendication 2, dans lequel l'agent d'inoculation est distribué parmi des tests en réplicat, à chaque concentration d'antibiotique, de manière telle à obtenir un nombre défini d'unités formant des colonies dans chaque et dans tous les réplicats.

6. Procédé selon la revendication 5, dans lequel dans chaque test le nombre est dans la gamme de 25-500, de préférence de 100-200.

7. Procédé selon la revendication 5 ou 6, dans lequel le nombre de réplicats est d'entre 20-100, typiquement d'environ 50.

8. Procédé selon la revendication 1, dans lequel une observation de la croissance est faite à environ 5 heures d'incubation.

9. Procédé selon la revendication 1, dans lequel des observations de croissance sont faites entre 6-12 heures.

10. Procédé selon la revendication 1, dans lequel des observations de croissance sont faites entre 18-24 heures.

11. Procédé selon la revendication 1, dans lequel des observations périodiques semi-continues, ou une surveillance continue, sont effectuées pendant 18-24 heures au moins afin d'évaluer automatiquement les modèles de croissance.

12. Procédé selon la revendication 11, dans lequel les premiers résultats sont présentés dès que des effets différentiels significatifs sont détectés.

13. Procédé selon la revendication 2, dans lequel la différenciation dans la réponse de croissance des populations microbiennes indique une population de microbes pharmacorésistants.

14. Procédé selon la revendication 3, dans lequel la différenciation dans la réponse de croissance des populations microbiennes indique une population de microbes dans un nutriment favorable à la croissance.

15. Procédé selon l'une quelconque des revendications précédentes, dans lequel la différenciation entre le groupe de population principale et le groupe de sous-population mineure, est déterminée et enregistrée, le procédé est répété et la différenciation entre les groupes de population est comparée à un ou plusieurs résultats précédents afin de déterminer s'il y a eu un changement quelconque dans une ou dans les deux populations microbiennes ou non.
